(19) Europäisches Patentamt
European Patent Office
Office européen des brevets

(11) **EP 3 467 371 A1**

(12) **EUROPEAN PATENT APPLICATION**

(43) Date of publication:
**10.04.2019 Bulletin 2019/15**

(51) Int Cl.:
*F21K 2/00* *(2006.01)* *C12M 1/00* *(2006.01)*
*C12M 3/00* *(2006.01)*

(21) Application number: **17382660.3**

(22) Date of filing: **04.10.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Valeo Iluminacion**
**23600 Martos (ES)**

(72) Inventors:
• **MAYOR, Raquel**
**23600 MARTOS (ES)**

• **PARRAS, Juan-Carlos**
**23600 MARTOS (ES)**
• **ORISICH, John**
**SEYMOUR, OH Ohio 47274 (US)**
• **CALAIS, Valère**
**SEYMOUR, OH Ohio 47274 (US)**

(74) Representative: **Oggioni, Baptiste**
**Valeo Vision S.A.S.**
**34, rue Saint-André**
**93012 Bobigny Cedex (FR)**

(54) **LIGHTING DEVICE**

(57) The invention is related to a lighting device for an automotive vehicle. This lighting device comprises a main container, a culture medium, a pumping circuit and filtering means. The main container houses a bioluminescent light source, the bioluminescent light source comprising bioluminescent living organisms suitable for emitting light. The culture medium is adapted for providing the bioluminescent living organisms with adequate conditions to live. The pumping circuit is suitable for recirculating the culture medium. The filtering means for removing waste products from the main container.

FIG.1

**Description**

**TECHNICAL FIELD**

This invention is related to the field of automotive lighting devices.

**STATE OF THE ART**

A lighting device comprises a light source, so that the lighting device may provide some light. Several types of light sources families are used nowadays, all of them having advantages and disadvantages.

Semiconductor light sources, such as Light Emitting Diodes (LEDs) or laser light sources have been used due to their efficiency and have a great improvement margin.

However, this invention is related to an alternative way of providing lighting in an automotive vehicle.

**DESCRIPTION OF THE INVENTION**

The invention provides a solution for this problem by means of a lighting device according to claim 1. Preferred embodiments of the invention are defined in dependent claims.

In an inventive aspect, the invention provides a lighting device for an automotive vehicle, the lighting device comprising

> a main container with a bioluminescent light source, the bioluminescent light source comprising bioluminescent living organisms suitable for emitting light;
> a culture medium adapted for providing the bioluminescent living organisms with adequate conditions to live;
> pumping circuit for recirculating the culture medium;
> filtering means for processing waste products from the main container.

Advantageously, this lighting device does not require electric power to emit light, hence being an alternative way of providing light in an automotive vehicle.

Advantageously, the bioluminescent living organisms comprise luciferin type molecules and luciferase enzyme. Advantageously, the luciferin type molecules are suitable to be oxidized by the luciferase enzyme to produce oxyluciferin molecules, such reaction causing an emission of photons and a production of waste products.

In some particular embodiments, the living organisms comprise bacteria, such as Vibrio Fischeri, Vibrio Harveyi or Photobacterium Phosphoreum. In other particular embodiments, the living organisms comprise dinoflagellates, such as Pyrocystis Fusiformis, Pyrocystis Noctiluca or Pyrocystis Lunula.

These types of living organisms are suitable for being used in this application, since they are bioluminescent, and emit light just with the only condition that they are provided with a suitable medium to stay alive.

Advantageously, such medium can comprise at least oxygen ($O_2$). If needed, the medium can also comprise activation molecules such as adenosine triphosphate (ATP) or sugar molecules, and/or minerals such as magnesium ion $Mg^{2+}$ or calcium ion.

When provided with the appropriate medium, the luciferase enzyme catalyses the oxidation of the luciferin molecules (L) to produce oxyluciferin (oxy-L). The oxyluciferin, being unstable, turns to luciferin with releasing light energy as photons (hv), and waste product as carbon dioxide ($CO_2$). The reaction can also release other waste products, such as adenosine monophosphate (AMP) and phosphate compounds, such as pyrophosphate PPi.

For instance, this reaction may be shown as follows:

$$L + O_2 + ATP \xrightarrow[Mg^{2+}]{luciferase} oxy - L + CO_2 + AMP + PP_i + hv$$

Advantageously, bioluminescent living organisms also comprise a luciferin-regenerating enzyme (LRE), suitable to improve the regeneration of the luciferin type molecules from the oxyluciferin molecules. After several cycles of oxidation and regeneration reactions, the luciferin type molecules die, becoming also waste products.

In some particular embodiments, the pumping circuit comprises

> a culture container, containing culture medium;
> a waste container, for storing waste products;

a first pump adapted to create a first flow from the culture container to the main container, this first flow containing culture medium; and

a second pump adapted to create a second flow from the main container to the waste container, this second flow containing culture medium, living organisms and waste products.

These first and second pumps are suitable for providing an equilibrium in the amount of culture medium, living organisms and waste products in the main container, where the bioluminescent light source is stored.

In some particular embodiments, the lighting device advantageously comprises a third pump adapted to create a third flow from the waste container to the culture container, this third flow containing waste products.

This third flow is particularly beneficious since it allows the circular communication between all the containers involved in the lighting device.

In other particular embodiments, the culture container and the waste container are the same container.

These embodiments provide a simpler pumping circuit, since the first and second pumps are enough to provide the circular communication between all the containers involved in the lighting device. The filtering means are therefore suitable to process at least some of the waste products so as to produce at least a part of the culture medium.

In some particular embodiments, the lighting device further comprises control means adapted to control the first pump and the second pump.

This control means, such as an Arduino, may adapt the operation of the first and second pumps to the growing rate of the living organisms.

In some particular embodiments, the lighting device further comprises an inner conduit adapted to feed the culture container from an auxiliary device of the automotive vehicle.

This makes possible that the culture medium is provided to the culture container in a different location from the lighting device itself, and is then conveyed by the inner conduit. The culture container may be therefore hidden, making the lighting device suitable for a wider range of applications.

In some particular embodiments, the filtering means comprises a plurality of sterilization filters arranged in an input port and an output port of the main container.

These sterilization filters thus arranged isolate the main container from contamination which may affect to the bioluminescent light source performance.

In some particular embodiments, the filtering means comprise a plurality of sterilization laser devices arranged in an input port and an output port of the main container.

The aim of this sterilization lasers is to avoid contamination completely.

In some particular embodiments, the filtering means comprises photosynthetic organisms, such as chloroplasts, suitable to convert by photosynthesis at least some of the waste products of the luciferin oxidation, such as the carbon dioxide ($CO_2$) into at least a part of the culture medium, such as the oxygen ($O_2$) and the adenosine triphosphate (ATP).

In these embodiments, the filtering means further comprises a filtering device adapted to remove part of the waste products from the culture container.

In the case where such photosynthesis reaction requires light, the waste container containing the filtering means can be placed in such a way the filtering means are exposed to ambient light during daytime, e.g., comprising a transparent portion. In such a case, the lighting device operates in a day/night cycle wherein:

- during daytime, the culture medium is generated by the filtering means through a photosynthesis reaction from the wasted products of the luciferin oxidation and from the ambient light, and
- during nightime, the culture medium is consumed by the bioluminescent living organisms through an oxidation of its luciferin molecules to produce light.

In another embodiment, the filtering means can comprise a light source suitable to produce the amount of light required by the photosynthesis reaction.

Advantageously, at least one of the containers, and especially each of the containers, is provided with an opening for replacing its content.

In some particular embodiments, at least one of the main container, the culture container and the waste container comprises openings, so that its content may be replaced.

In some particular embodiments, the lighting device further comprises optical means configured to project light emitted by the bioluminescent light source.

In the event that the light emitted by the bioluminescent light source may be used for lighting, the optical means may modify this emitted light to have desired features.

In some particular embodiments, the lighting device further comprises interrupting means for blocking the light emission from the bioluminescent light source.

This blocking may be useful when the lighting device is used in a function that may need to be put off in some particular situations. Since the bioluminescent living organisms emit light continuously, a way of achieving this "put-off effect" is blocking this emission of light, so that it is not visible from the exterior.

In some particular embodiments, the interrupting means comprises a plate adapted for being removably arranged covering the main container so that when the plate covers the bioluminescent light source, the bioluminescent light source is not seen.

This is an easy way of interrupting the light emission outside the lighting device. In this case, the bioluminescent light source is not moved, and an external element, such as a plate, is interposed between the bioluminescent light source and the ambient to be lighted.

In some particular embodiments, the interrupting means an opaque element covering a first portion of the main container, and spinning means adapted to turn the main container between an invisible position, where at least part of the first portion is interposed between the bioluminescent light source and an exterior zone, and a visible position, where the bioluminescent light source is seen from the exterior zone.

This alternative is a more sophisticated way of interrupting the light emission outside the lighting device. In this case, there is not any additional element which is arranged or removed, but the same bioluminescent light source always comprises an opaque element. However, this opaque element only covers a first portion of the bioluminescent light source. In the visible position, the bioluminescent light source is oriented such that the opaque element is not interposed between the living organisms and the exterior zone, while in the invisible position, the bioluminescent light source is oriented such that at least part of the opaque element is interposed between the living organisms and the outside, so that no light is perceived from the exterior.

In some particular embodiments, the lighting device is an ambient lighting device.

## BRIEF DESCRIPTION OF THE DRAWINGS

To complete the description and in order to provide for a better understanding of the invention, a set of drawings is provided. Said drawings form an integral part of the description and illustrate an embodiment of the invention, which should not be interpreted as restricting the scope of the invention, but just as an example of how the invention can be carried out. The drawings comprise the following figures:

Figure 1 shows an automotive vehicle with an ambient lighting device according to the invention.

Figures 2a and 2b shows an embodiment of a lighting device comprising interrupting means.

Figures 3a and 3b shows another embodiment of a lighting device comprising interrupting means.

Fig.4 shows an other embodiment of a lighting device according to the invention.

## DETAILED DESCRIPTION OF THE INVENTION

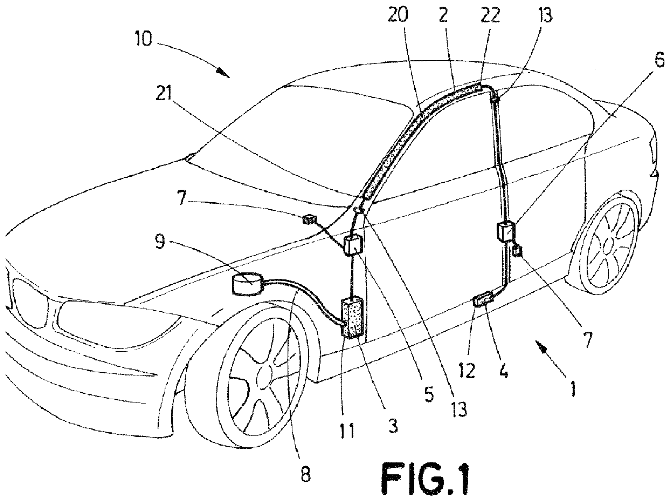

Figure 1 shows an automotive vehicle (10) with an ambient lighting device (1) according to the invention.
Such a lighting device comprises

a main container (2) with a bioluminescent light source, the bioluminescent light source comprising bioluminescent living organisms (20) suitable for emitting light;
a culture container (11) with a culture medium (3), the culture medium (3) being adapted for providing the bioluminescent living organisms (20) with adequate conditions to live;
a waste container (12), for storing waste products (4);
a first pump (5) adapted to create a first flow from the culture container (11) to the main container (2), this first flow containing culture medium (3); and
a second pump (6) adapted to create a second flow from the main container (2) to the waste container (12), this second flow containing culture medium (3), living organisms (20) and waste products (4).
filtering means for removing waste products (4) from the main container (2).

The first pump (5) and the second pump (6) are controlled by control means (7).
The first pump (5) is intended to pump culture medium (3) into the main container (2), so that the living organisms (20) may be fed. The second pump (6) is intended to extract some of the content of the main container (2), including living organisms (20), waste products (4) and culture medium (3). This content is filtered so that the waste products (4) are sent to the waste container (12) and the rest of the content is sent to the culture container (11). The filtering means comprises a plurality of sterilization filters (13) arranged in an input port (21) and an output port (22) of the main container (2). In other embodiments, the filtering means comprise a plurality of sterilization laser devices arranged in an input port and an output port of the main container. The filter in the output port (22) of the main container (2) filters the waste products, which are sent to the waste container (12). The filter in the input (21) of the main container (2) filters the waste products which may be present in the culture container (11), either because they result of a reaction occured after passing the filter in the output (22) of the main container (2) or because they have escaped from the filter in the output (22) of the main container (2).

In the present embodiment, the living organisms (20) comprise bacteria, such as Vibrio Fischeri, Vibrio Harveyi or Photobacterium Phosphoreum. However, in different embodiments, other suitable living organisms (20) may be used.

As may be seen in this figure, the lighting device (1) further comprises an inner conduit (8) adapted to feed the culture container (11) from an auxiliary device (9) of the automotive vehicle (10).

In some embodiments, the bioluminescent light source is used for signalling, so the lighting device also comprises optical means, such as a collimator or a reflector, to project a light beam outside such lighting device.

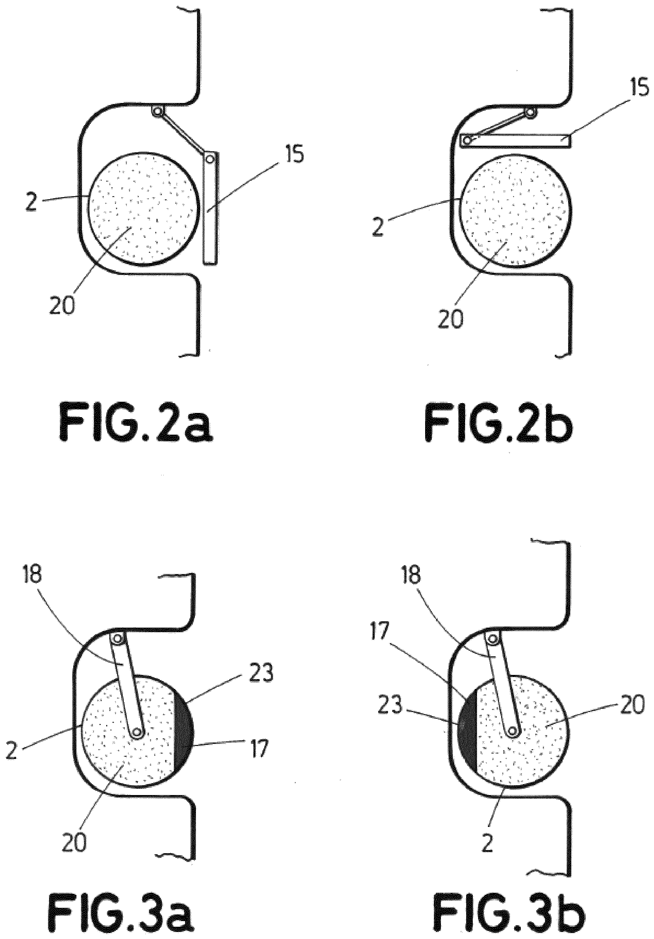

Figures 2a and 2b shows an embodiment of a lighting device comprising interrupting means, which are some elements intended to block the pass of light from the bioluminescence light source to the exterior.

In this case, the interrupting means comprises a plate (15), adapted for being removably arranged covering the main container (2) so that when the plate (15) covers the bioluminescent light source, the bioluminescent light source is not seen.

Figure 2a shows the plate in the position covering the main container, so that the bioluminescent light source is not seen. Figure 2b shows the plate in the position letting the light from the bioluminescent light source exit freely, so that it is seen.

Figures 3a and 3b shows another embodiment of a lighting device comprising interrupting means.

In this case, the interrupting means comprises an opaque element (17) covering a first portion (23) of the main container (2), and spinning means (18) adapted to turn the main container (2) between an invisible position, where at least part of the first portion (23) is interposed between the bioluminescent light source and an exterior zone, and a visible position, where the bioluminescent light source is seen from the exterior zone.

Figure 3a shows the main container in the invisible position, so that the bioluminescent light source is not seen. Figure 3b shows the plate in the visible position, letting the light from the bioluminescent light source exit freely, so that it is seen.

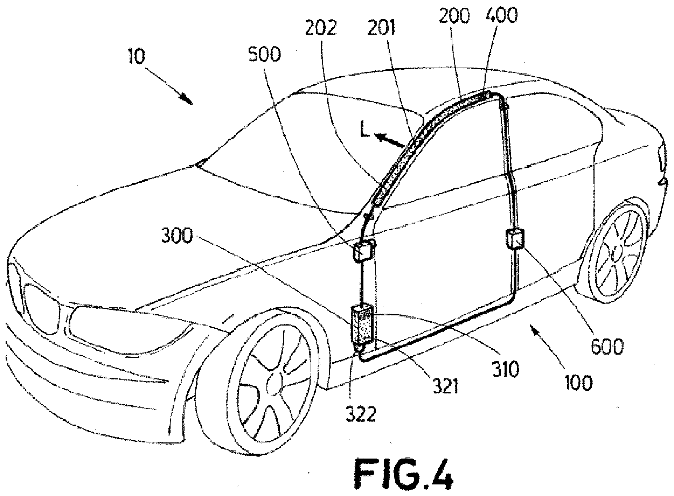

Figure 4 shows another embodiment of a lighting device (100). Such a lighting device (100) comprises

a main container (200) with a bioluminescent light source, the bioluminescent light source comprising bioluminescent living organisms (201) suitable for emitting light;
a culture container (300) comprising:

a culture medium (310), the culture medium (310) being adapted for providing the bioluminescent living organisms (201) with adequate conditions to live;
filtering means comprising photosynthetic organisms (321) suitable to convert a part of the waste products (400) from the main container (200) into a part of the culture medium (310) and a filtering device (322) adapted to remove the other part of the waste products (400) from the culture container (300);

a first pump (500) adapted to create a first flow from the culture container (310) to the main container (200), this first flow containing culture medium (310); and
a second pump (600) adapted to create a second flow from the main container (200) to the culture container (300), this second flow containing waste products (400).

In the present embodiment, the living organisms (201) comprise Lampyris luciferin type molecules, such as Photinus pyralis, luciferase enzyme and luciferin-regenerating enzyme.

When provided by the first pump (500) with the medium (310) comprising water, oxygen ($O_2$), adenosine triphosphate (ATP) and magnesium ion $Mg^{2+}$, the luciferase enzyme catalyzes the oxidation of the luciferin molecules to produce oxyluciferin molecules (202) with releasing photons (L).

The reaction also releases waste products (400) comprising water, carbon dioxide ($CO_2$), adenosine monophosphate (AMP) and phosphate compounds, such as pyrophosphate $PP_i$. The luciferin-regenerating enzyme improves the regeneration of the luciferin type molecules from the oxyluciferin molecules, until the luciferin type molecules die, becoming therefore also a waste product (400).

The photosynthetic organisms (321) comprise chloroplasts suitable to convert a part of the waste products (400), such as the carbon dioxide ($CO_2$) and the water, pumped from the main container (200) by the second pump (600), into a part of the culture medium (310), such as the oxygen ($O_2$), the water and the adenosine triphosphate (ATP).

The filtering device (322) conform with the filtering means of Figure 1, removes the other part of the waste products (400), such as the dead luciferin molecules from the culture container (300).

The culture container (300) is provided with an opening so as to fill it with a part of the medium (310) that is not generated by the photosynthetic organisms (321), such as the magnesium ion.

The housing of the culture container (300) is also provided with a transparent part, placed in the vehicle so as to permit to ambient light to enter in the culture container (300) in such a way that it allows the photosynthetic organisms (321) to perform photosynthesis.

**Claims**

1. Lighting device (1) for an automotive vehicle (10), the lighting device (1) comprising

   a main container (2) with a bioluminescent light source, the bioluminescent light source comprising bioluminescent living organisms (20) suitable for emitting light;
   a culture medium (3) adapted for providing the bioluminescent living organisms (20) with adequate conditions to live;
   pumping circuit for recirculating the culture medium (3);
   filtering means for processing waste products (4) from the main container (2).

2. Lighting device (1) according to claim 1, wherein the living organisms (20) comprise luciferin type molecules and luciferase enzyme.

3. Lighting device (1) according to claim 2, wherein the living organisms (20) comprise bacteria, such as Vibrio Fischeri, Vibrio Harveyi or Photobacterium Phosphoreum.

4. Lighting device (1) according to claim 2, wherein the living organisms (20) comprise dinoflagellates, such as Pyrocystis Fusiformis, Pyrocystis Noctiluca or Pyrocystis Lunula.

5. Lighting culture medium comprises at least one of oxygen, adenosine triphosphate, calcium ions and magnesium ions.

6. Lighting device (1) according to any of the preceding claims, wherein the pumping circuit comprises

   a culture container (11), containing culture medium (3);
   a waste container (12), for storing waste products (4);
   a first pump (5) adapted to create a first flow from the culture container (11) to the main container (2), this first flow containing culture medium (3); and
   a second pump (6) adapted to create a second flow from the main container (2) to the waste container (12), this second flow containing culture medium (3), living organisms (20) and waste products (4).

7. Lighting device (1) according to any one of the preceding claims, further comprising a third pump (51) adapted to create a third flow from the waste container (12) to the culture container (11), this third flow containing waste products (4).

8. Lighting device (1) according to any one of claims 1 to 6, wherein the culture container (11) and the waste container (12) are the same container.

9. Lighting device (1) according to any of claims 6 to 8, further comprising an inner conduit (8) adapted to feed the culture container (11) from an auxiliary device (9) of the automotive vehicle (10).

10. Lighting device (1) according to any of claims 6 to 9, wherein

    the filtering means comprises photosynthetic organisms, such as chloroplasts, suitable to convert by photosynthesis at least some of the waste products into at least a part of the culture medium;
    the filtering means further comprises a filtering device adapted to remove part of the waste products from the culture container; and
    the culture container contains a transparent portion, so that ambient light may reach its content.

11. Lighting device (1) according to any of claims 6 to 10, wherein at least one of the main container, the culture container and the waste container comprises openings, so that its content may be replaced.

12. Lighting device (1) according to any of claims 1 to 9, wherein the filtering means comprise a plurality of sterilization filters (13) arranged in an input port (21) and an output port (22) of the main container (2).

13. Lighting device (1) according to any of claims 1 to 9, wherein the filtering means comprise a plurality of sterilization laser devices arranged in an input port and an output port of the main container.

14. Lighting device (1) according to claim 16, wherein the interrupting means comprises a plate (15) adapted for being removably arranged covering the main container (2) so that when the plate (15) covers the bioluminescent light source, the bioluminescent light source is not seen.

15. Lighting device according to any of the preceding claims, wherein the interrupting means comprises an opaque element (17) covering a first portion (23) of the main container (2), and spinning means (18) adapted to turn the main container (2) between an invisible position, where at least part of the first portion (23) is interposed between the bioluminescent light source and an exterior zone, and a visible position, where the bioluminescent light source is seen from the exterior zone.

**FIG.1**

FIG.2a

FIG.2b

FIG.3a

FIG.3b

**FIG.4**

Europäisches
Patentamt

European
Patent Office

Office européen
des brevets

# EUROPEAN SEARCH REPORT

Application Number

EP 17 38 2660

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Eduardo Mayoral: "Bioluminescent devices for zero-electricity lighting, Seville, Spain", , 2011, XP002778285, Retrieved from the Internet: URL:http://epiteszforum.hu/files/HA11_EUR_NG3_WinnerPoster.pdf [retrieved on 2018-02-16] * the whole document * | 1-15 | INV. F21K2/00 C12M1/00 C12M3/00 |
| A | WO 2007/101434 A2 (FRITZMEIER GEORG GMBH & CO KG [DE]; UPHOFF CHRISTIAN [DE]) 13 September 2007 (2007-09-13) * the whole document * | 1-15 | |
| A | Lourdes Riquelme: "Bioluminescent Bacteria and Algae, the Future Lighting", , 12 March 2015 (2015-03-12), XP002778286, Retrieved from the Internet: URL:https://labiotech.eu/bioluminescent-bacteria-and-algae-lighting/ [retrieved on 2018-02-15] * the whole document * | 1-15 | |
| | | | TECHNICAL FIELDS SEARCHED (IPC) F21K C12M |
| A | DATABASE WPI Week 201472 Thomson Scientific, London, GB; AN 2014-N97771 XP002778287, & IN MUM 201203508 A (LOKAPURE S G) 20 June 2014 (2014-06-20) * the whole document * | 1-15 | |
| A | US 5 554 035 A (GOOCH VAN D [US]) 10 September 1996 (1996-09-10) * the whole document * | 1-15 | |

-/--

The present search report has been drawn up for all claims

| Place of search | Date of completion of the search | Examiner |
|---|---|---|
| The Hague | 19 February 2018 | Smalt, Rolf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 3 467 371 A1

Europäisches
Patentamt

European
Patent Office

Office européen
des brevets

# EUROPEAN SEARCH REPORT

Application Number

EP 17 38 2660

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 3 674 648 A (SOLI GIORGIO)<br>4 July 1972 (1972-07-04)<br>* the whole document * | 1-15 | |
| A | ES 2 498 739 A2 (UNIV SEVILLA [ES])<br>25 September 2014 (2014-09-25)<br>* the whole document * | 1-15 | |
| A | ES 2 497 340 A1 (UNIV SEVILLA [ES])<br>22 September 2014 (2014-09-22)<br>* the whole document * | 1-15 | |
| A | Teresa van Dongen: "Ambio, bacterial lamp by Teresa van Dongen",<br>,<br>2014, XP002778288,<br>Retrieved from the Internet:<br>URL:http://teresavandongen.com/ambio<br>[retrieved on 2018-02-01]<br>* the whole document * | 1-15 | |
| A | Randolph Jonsson: "Philips Bio-light concept lights the home using bacteria",<br>,<br>28 November 2011 (2011-11-28),<br>XP002778289,<br>Retrieved from the Internet:<br>URL:https://newatlas.com/philips-bio-light-concept-taps-bioluminescence-for-home-use/20632/<br>[retrieved on 2018-02-15]<br>* the whole document * | 1-15 | TECHNICAL FIELDS SEARCHED (IPC) |

The present search report has been drawn up for all claims

| Place of search | Date of completion of the search | Examiner |
|---|---|---|
| The Hague | 19 February 2018 | Smalt, Rolf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

12

## ANNEX TO THE EUROPEAN SEARCH REPORT
## ON EUROPEAN PATENT APPLICATION NO.

EP 17 38 2660

This annex lists the patent family members relating to the patent documents cited in the above-mentioned European search report.
The members are as contained in the European Patent Office EDP file on
The European Patent Office is in no way liable for these particulars which are merely given for the purpose of information.

19-02-2018

| Patent document cited in search report | | Publication date | Patent family member(s) | | Publication date |
|---|---|---|---|---|---|
| WO 2007101434 | A2 | 13-09-2007 | EP | 1991687 A2 | 19-11-2008 |
| | | | EP | 2322639 A1 | 18-05-2011 |
| | | | US | 2009130256 A1 | 21-05-2009 |
| | | | WO | 2007101434 A2 | 13-09-2007 |
| IN MUM201203508 | A | 20-06-2014 | | | |
| US 5554035 | A | 10-09-1996 | NONE | | |
| US 3674648 | A | 04-07-1972 | NONE | | |
| ES 2498739 | A2 | 25-09-2014 | ES | 2497340 A1 | 22-09-2014 |
| | | | ES | 2498739 A2 | 25-09-2014 |
| ES 2497340 | A1 | 22-09-2014 | ES | 2497340 A1 | 22-09-2014 |
| | | | ES | 2498739 A2 | 25-09-2014 |

EPO FORM P0459

For more details about this annex : see Official Journal of the European Patent Office, No. 12/82